(19) Europäisches Patentamt
European Patent Office
Office européen des brevets

(11) Veröffentlichungsnummer: **0 311 893 B1**

(12) **EUROPÄISCHE PATENTSCHRIFT**

(45) Veröffentlichungstag der Patentschrift: **30.12.92**

(51) Int. Cl.$^5$: **C07C 221/00**

(21) Anmeldenummer: **88116526.0**

(22) Anmeldetag: **06.10.88**

# (54) Verfahren zur Herstellung von 2,5-Bis-(aminoarylamino)-benzochinonen.

(30) Priorität: **16.10.87 DE 3735093**

(43) Veröffentlichungstag der Anmeldung:
**19.04.89 Patentblatt 89/16**

(45) Bekanntmachung des Hinweises auf die Patenterteilung:
**30.12.92 Patentblatt 92/53**

(84) Benannte Vertragsstaaten:
**CH DE FR GB LI**

(56) Entgegenhaltungen:
**EP-A- 0 171 811**
**EP-A- 0 245 603**
**FR-A- 580 007**
**FR-A- 798 447**

(73) Patentinhaber: **BAYER AG**
**W-5090 Leverkusen 1 Bayerwerk(DE)**

(72) Erfinder: **Stawitz, Josef-Walter, Dr.**
**Am Hagen 1**
**W-5068 Odenthal-Gloebusch(DE)**

EP 0 311 893 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der vorliegenden Erfindung ist ein Verfahren zur Herstellung von 2,5-Bis-(aminoarylamino)-benzochinonen (I), dadurch gekennzeichnet, daß man 2 Mol eines Aminoarylamins (II) mit 1 Mol eines gegebenenfalls substituierten Benzochinons (III) bzw. Hydrochinons (IV) in Gegenwart von Oxidationsmitteln umsetzt.

Im Unterschied hierzu handelt es sich bei dem aus EP-A-245 603 bekannten Verfahren um eine Kondensationsreaktion zwischen einem Chinon und einer Diaminoverbindung.

Die Umsetzung erfolgt vorzugsweise in wäßrigem oder wäßrig-organischem Medium bei Temperaturen von etwa 10 bis 120 ° C, vorzugsweise 30 bis 70 ° C, vorzugsweise im Molverhältnis 2:1.

Geeignete Oxidationsmittel sind beispielsweise Chlorate, Iodate, Bromate, Persulfate, Peroxidisulfate, insbesondere die entsprechenden Alkalisalze (Na, K, Li), ferner $H_2O_2$ und insbesondere Sauerstoff (Luft).

Vorzugsweise wird in Gegenwart von Katalysatoren wie Kupfer-, Mangan-, Eisen-III- oder Vanadiumverbindungen gearbeitet, wobei im allgemeinen etwa 0,1 bis 2 Gew.-% Katalysator, bezogen auf (II) zur Anwendung gelangen.

Geeignete Katalysatoren sind beispielsweise $MnSO_4$, $MnO_2$, Fe-III-Salze, Anthrachinon-2-sulfonsäure sowie bevorzugt Vanadiumverbindungen wie $NaVO_3$, $NH_4VO_3$ oder $V_2O_5$.

In allgemeinen werden 1 bis 3 Mol Oxidationsmittel pro Mol (IV) bzw. (III) eingesetzt.

Geeignete, gegebenenfalls mitzuverwendende Lösungsmittel sind insbesondere wasserlösliche Alkohole oder Ketone wie Methanol, Ethanol, Isopropanol, Aceton.

Bei Verwendung von Sauerstoff (Luft) als Oxidationsmittel arbeitet man vorzugsweise in Abwesenheit von Säuren, bei Verwendung anderer Oxidationsmittel hingegen vorzugsweise in Gegenwart von Säuren wie Salzsäure, Schwefelsäure, Phosphorsäure, Essigsäure, Propionsäure, d.h. im pH-Bereich von etwa 1 bis 4.

Vorzugsweise setzt man Hydrochinone der Formel

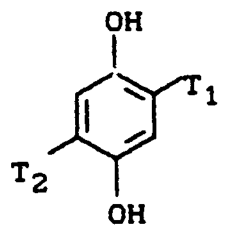

(V)

worin

$T_1$, $T_2$ unabhängig voneinander H, Halogen (Cl, Br), $SO_3H$, COOH, COOR (R = $C_1$-$C_4$-Alkyl), $C_1$-$C_4$-Alkoxy, $C_1$-$C_4$-Alkyl, Phenyl, Phenoxy bedeuten

mit Aminoarylaminen der Formel

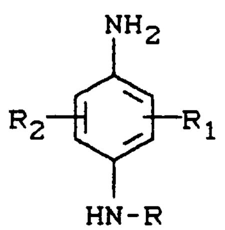

(VI)

um, worin

R H, Acyl, insbesondere $C_1$-$C_6$-Alkylcarbonyl oder Phenylcarbonyl, einen aliphatischen oder aromatischen Rest insbesondere $C_1$-$C_6$-Alkyl oder Phenyl bedeutet

$R_1$, $R_2$ unabhängig voneinander H, Halogen (Cl, Br), $SO_3H$, COOH, $NO_2$, $C_1$-$C_6$-Alkyl, $C_1$-$C_6$-Alkoxy, $C_1$-$C_6$-Alkylcarbonylamino, $C_1$-$C_6$-Alkylamino, $C_1$-$C_6$-Alkylsulfonyl, Phenyl, Phenoxy bedeuten

um, wobei Verbindungen der Formel

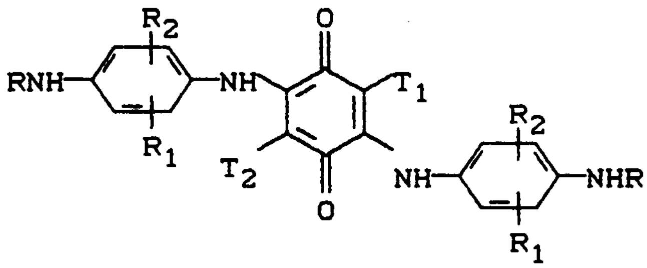

(VII)

erhalten werden.

Die oben erwähnten Alkyl-, Phenyl-, Alkoxy- und Phenoxyreste können weitere übliche Substituenten aufweisen, beispielsweise Halogen (Cl, Br), OH, $SO_3H$, COOH, $NH_2$, $NO_2$, $C_1$-$C_4$-Alkoxy, $C_1$-$C_4$-Alkylthio, $C_1$-$C_4$-Alkylamino, $OSO_3H$, $CONH_2$, $SO_2NH_2$, Alkylamino, Phenylcarbonylamino, $C_1$-$C_4$-Alkylsulfonyl, $C_1$-$C_4$-Alkyl.

Setzt man eine Mischung verschiedener Verbindungen (VI) ein, erhält man im allgemeinen statistische Gemische der entsprechenden symmetrischen Verbindungen mit den entsprechenden unsymmetrischen Verbindungen.

Geeignete Verbindungen sind beispielsweise:

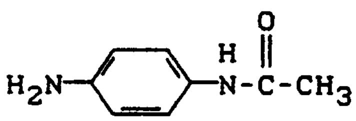 , 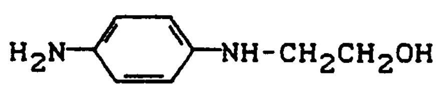

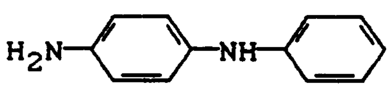

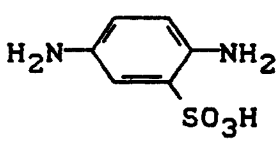

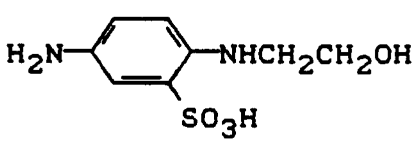

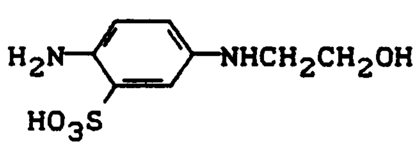

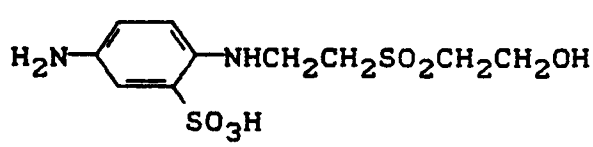

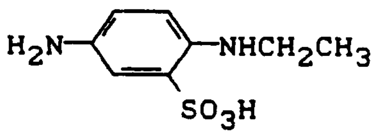
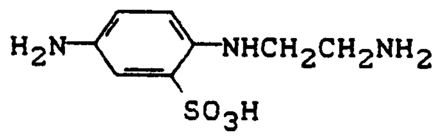

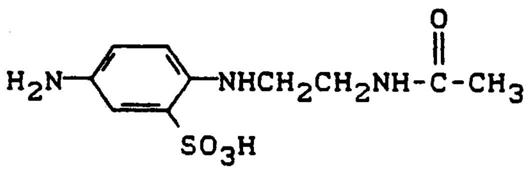

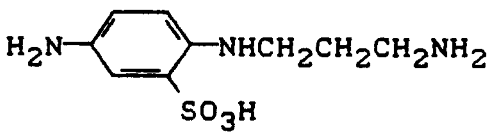

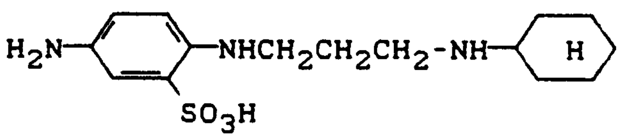

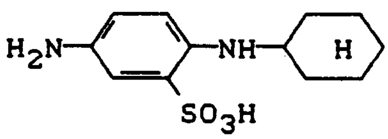
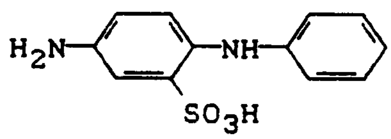

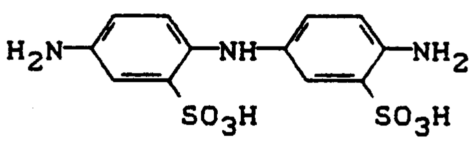
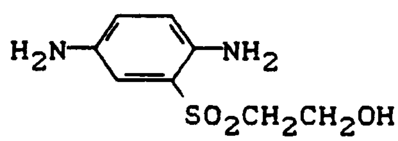

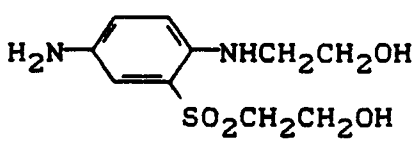
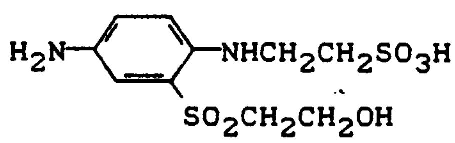

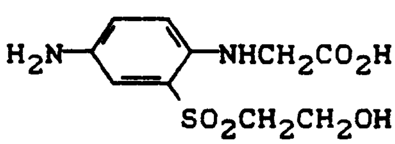
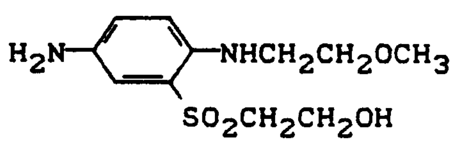

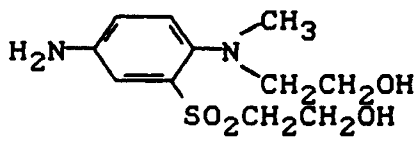

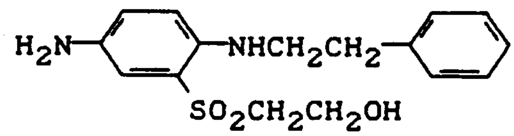

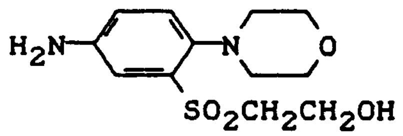

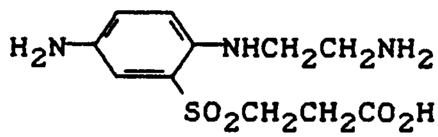

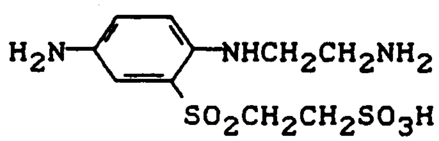

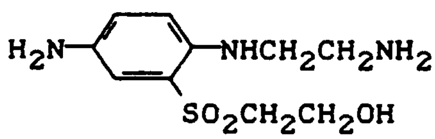

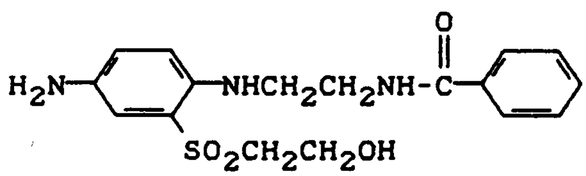

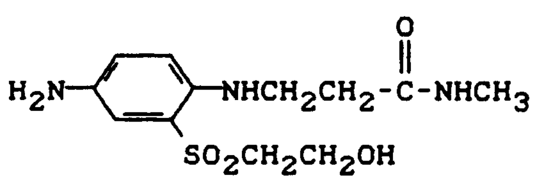

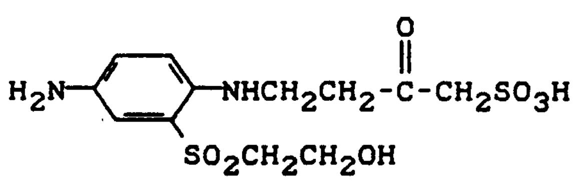

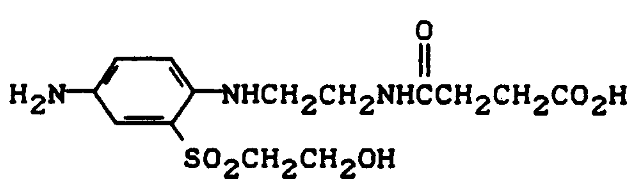

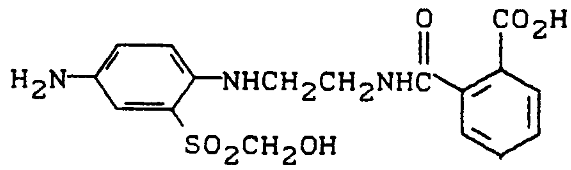

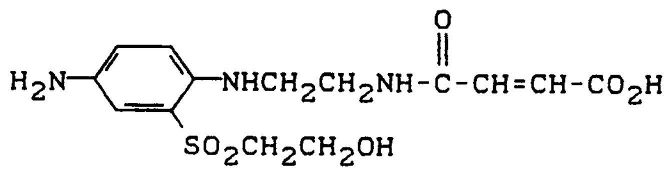

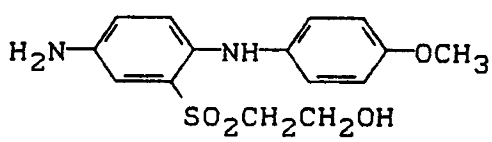

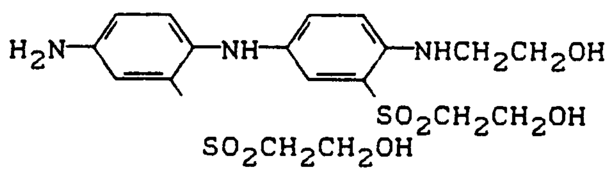

Diese Verbindungen sind bekannt bzw. lassen sich nach üblichen Verfahren herstellen, bspw. durch Umsetzung der entsprechenden substituierten 1-Chlor-4-Nitroaniline mit den entsprechenden Aminen und folgende Reduktion der Nitrogruppe zur Aminogruppe oder aber durch Umsetzung der entsprechenden Sulfinsäuren mit Ethylenoxid oder Chlorethanol (vgl. EP-A 153 599; 171 611; DE-A 1 644 663; US-PS 2 434 150, EP-A 258 493; EP-A 258 686).

Geeignete Verbindungen (V) sind insbesondere Hydrochinon.

Die Verbindungen (I) bzw. (VII) sind beispielsweise Ausgangsprodukte für die Herstellung von Triphendioxazinfarbstoffen, die man in bekannter Weise durch oxidativen Ringschluß erhält.

Beispiel 1

Zu einer Lösung von 5,8 g Hydrochinon und 26 g 4-$\beta$-Hydroxyethylamino-3-$\beta$-hydroxyethylsulfonylanilin in 310 ml 5 % Schwefelsäure werden bei 40 ° C 0,3 g $NaVO_3 \cdot 4H_2O$ gegeben, dann innerhalb von 20 Minuten eine Lösung aus 7,5 g $NaClO_3$ in 50 ml Wasser getropft. Zunächst durch leichte Kühlung, dann durch Heizen wird die Temperatur bei 40 bis 45 ° C gehalten. Nach 6 Stunden bei 40 bis 45 ° C läßt man auf Raumtemperatur abkühlen, saugt ab, wäscht mit Wasser bis zum farblosen Ablauf und trocknet bei 50 ° C im Vakuum. Man erhält 26,8 g eines dunklen Pulvers, das hauptsächlich aus Bisanil der folgenden Formel besteht.

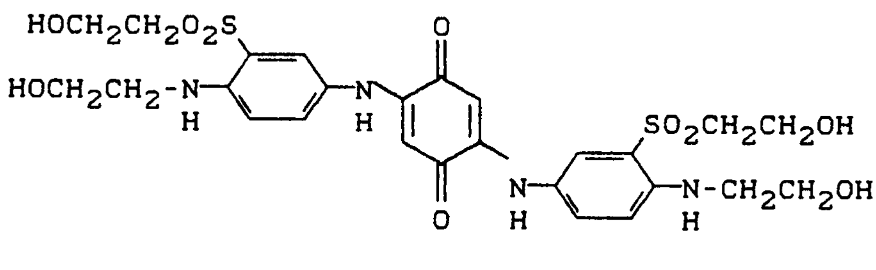

Beispiel 2

In 160 ml 10 %ige Schwefelsäure trägt man nacheinander 6,6 g Hydrochinon, 0,2 g $NaVO_3.4H_2O$ und 3,5 g $NaClO_3$ ein, und erwärmt auf 40-45 ° C. Innerhalb von ca. 15 Min. entsteht eine gelbe Suspension von Benzochinon. Dann läßt man innerhalb von ca. 15 Minuten unter leichter Kühlung eine 60 ° C heiße Lösung von 26.0 g 4-β-Hydroxyethylamino-3-β-hydroxyethylsulfonyl-anilin in 200 ml Wasser und 50 ml Isopropanol so zulaufen, daß sich eine Temperatur von ca. 50 ° C einstellt. Anschließend wird innerhalb von 10 Minuten eine Lösung von 4,5 g NaClO3 in 50 ml Wasser zugetropft. Man läßt bei einer Temperatur von ca. 45 ° C ausreagieren und saugt nach ca. 4 Stunden ab. Nach Waschen mit Wasser bis zum farblosen neutralen Ablauf und Trocknen bei 50 ° C im Vakuum erhält man 28,8 g Bisanil, identisch mit dem Produkt aus Beispiel 1.

Beispiel 3

Eine Mischung aus 26 g 4-β-Hydroxyethylamino-3-β-hydroxyethylsulfonylanilin, 6,0 g Hydrochinon und 0,4 g $NaVO_3 \cdot 4H_2O$ in 300 ml Wasser wird auf 80 bis 90 ° C geheizt und ein kräftiger Luftstrom durchgeleitet. Nach 16 bis 18 Stunden ist das Anilinderivat verschwunden. Man läßt auf 30 ° C abkühlen, saugt ab, wäscht mit Wasser und trocknet. Man erhält 25,1 g Bisanil, identisch mit dem Produkt aus Beispiel 1.

Beispiel 4

Eine Mischung aus 150 ml 10 %iger Schwefelsäure, 6,0 g Hydrochinon, 0,1 g $V_2O_5$ und 2,5 g $NaClO_3$ wird auf 40 ° C erwärmt und 20 Minuten bei 40-45 ° C gehalten. Dann gibt man rasch eine Lösung von 4,0 g $NaClO_3$ in 20 ml Wasser zu und läßt eine Lösung von 23,1 g 3-Amino-6-β-aminoethylamino-benzolsulfonsäure in 200 ml 2 %iger Schwefelsäure zulaufen. Man hält über ca. 4 Stunden eine Temperatur von 40-45 ° C, kühlt auf Raumtemperatur und saugt ab. Nach Waschen mit Wasser und Trocknen bei 70 ° C im Vakuum erhält man 23,4 g Produkt, das hauptsächlich aus Bisanil der folgenden Formel besteht.

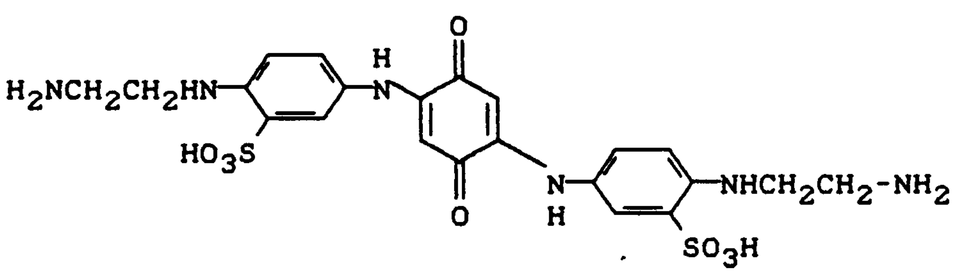

Beispiel 5

Zu einer Suspension von 5,8 g Benzochinon in 100 ml 20 %iger Schwefelsäure wird eine ca. 50 ° C warme Lösung von 36,5 g 3-β-Benzoylaminoethylamino-3-β-hydroxyethylsulfonylanilin in 200 ml Wasser und 100 ml Ethanol gegossen. Bei ca. 40 ° C tropft man innerhalb von 20 Minuten eine Lösung von 4,0 g $NaClO_3$ in 25 ml Wasser zu und hält über ca. 4 Stunden eine Temperatur von 45-50 ° C. Nach Erkalten wird abgesaugt, mit Wasser gewaschen und bei 70 ° C im Vakuum getrocknet. Man erhält 37,7 g eines dunklen Pulvers, das hauptsächlich aus Bisanil der folgenden Formel besteht.

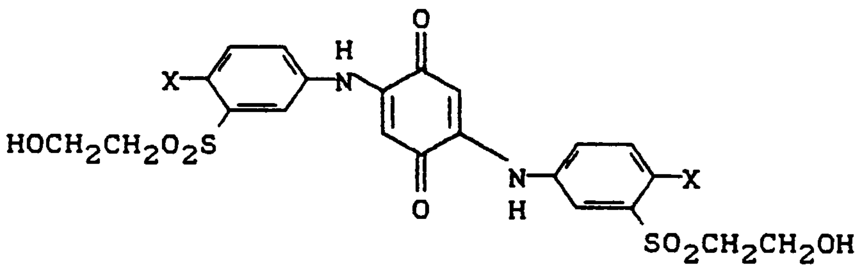

X = 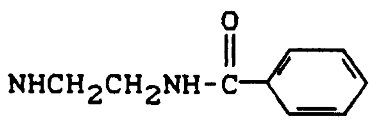

Beispiel 6

Zu einer Mischung aus 6,6 g Hydrochinon und 35,0 g 4-[β-(-Bernsteinsäuremonoamido)-ethylamino]-3-(β-hydroxyethylsulfonyl)-anilin in 300 ml 5 %iger Schwefelsäure und 50 ml Methanol werden 0,1 g $V_2O_5$ gegeben. Man heizt auf 40 ° C und tropft innerhalb von ca. 30 Minuten eine Lösung von 7,0 g $NaClO_3$ in 50 ml Wasser zu. Man hält ca. 5 Stunden eine Temperatur von 40-45 ° C, kühlt auf Raumtemperatur und saugt ab. Der Nutschkuchen wird mit Salzsäure-haltigem Wasser sulfatfrei gewaschen und bei 50 ° C im Vakuum getrocknet. Man erhält 33,1 g Produkt, das hauptsächlich aus Bisanil der folgenden Formel besteht.

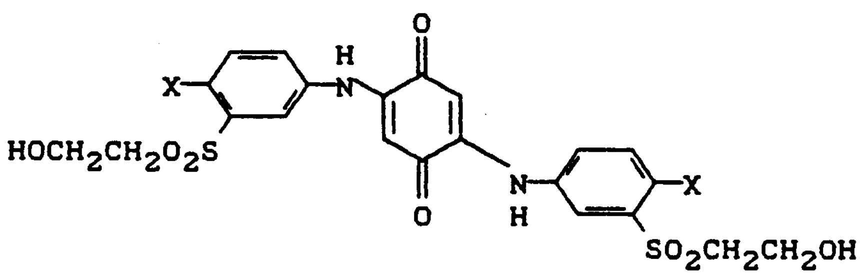

X = 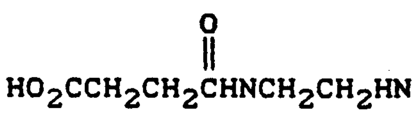

Beispiel 7

In 200 ml 10 %iger Schwefelsäure werden 6,6 g Hydrochinon, 11,6 g 3-Amino-6-β-hydroxyethylamino-benzolsulfonsäure, 13,0 g 4-β-Hydroxyethylamino-3-β-hydroxyethylsulfonyl-anilin und 0,2 g $NaVO_3.H_2O$ eingetragen. Nach Erwärmen auf 45 ° C wird innerhalb einer Stunde eine Lösung aus 7,0 g $NaClO_3$ in 150 ml Wasser zugetropft. Man hält noch 4 Stunden bei 40-45 ° C, kühlt auf Raumtemperatur und saugt ab. Der Nutschkuchen wird mit Wasser bis zum farblosen neutralen Ablauf gewaschen und bei 70 ° C im Vakuum getrocknet. Man erhält 23,1 g einer Mischung aus Bisanilen mit der Hauptkomponente.

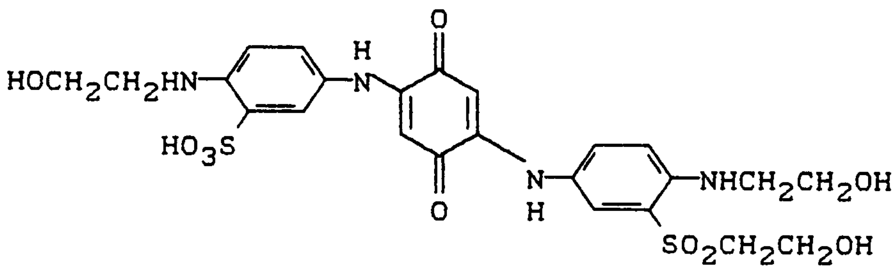

Beispiel 8

Eine Mischung aus 150 ml 10 %iger Schwefelsäure, 6,3 g Hydrochinon, 0,3 g $NaVO_3.4H_2O$ und 3,5 g $NaClO_3$ wird ca. 15 Minuten auf 40 °C erwärmt. Anschließend trägt man innerhalb von ca. 20 Minuten 17,2 g 87 %iges 4-Amino-acetanilid ein und tropft gleichzeitig eine Lösung von 3,0 g $NaClO_3$ in 20 ml Wasser zu. Man hält ca. 4 Stunden bei 40-45 °C, saugt ab, wäscht mit Wasser und heißem Methanol und trocknet bei 70 °C im Vakuum. Man isoliert 17,9 g Produkt, das hauptsächlich aus Bisanil der folgenden Formel besteht.

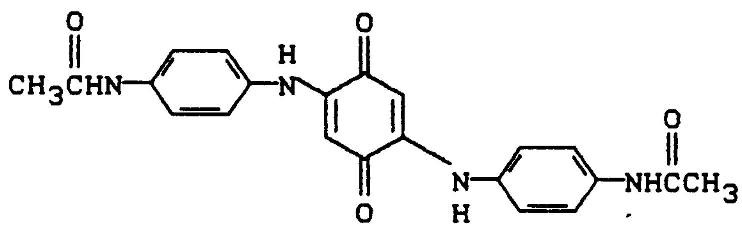

**Patentansprüche**

1. Verfahren zur Herstellung von 2,5-Bis-(aminoarylamino)-benzochinonen, dadurch gekennzeichnet, daß man 2 Mol eines Aminoarylamins mit 1 Mol eines Benzochinons bzw. Hydrochinons in Gegenwart von Oxidationsmitteln umsetzt.

2. Verfahren nach Anspruch 1, dadurch gekennzeichnet, daß man in wäßrigem oder wäßrig-organischem Medium bei etwa 10 bis 120 °C arbeitet.

3. Verfahren nach Anspruch 1, dadurch gekennzeichnet, daß man in wäßrig-saurem Medium arbeitet.

4. Verfahren nach Anspruch 3, dadurch gekennzeichnet, daß man in Gegenwart von $H_2SO_4$, $H_3PO_4$, HCl oder Essigsäure arbeitet.

5. Verfahren nach Anspruch 1, dadurch gekennzeichnet, daß man mit Chloraten, Bromaten oder Iodaten, gegebenenfalls in Gegenwart von Vanadiumverbindungen oxidiert.

6. Verfahren nach Anspruch 1, dadurch gekennzeichnet, daß man mit Luft in Gegenwart von Katalysatoren, insbesondere Eisen-, Kupfer-, Mangan- oder Vanadiumverbindungen oder Anthrachinon-2-sulfonsäure arbeitet.

7. Verfahren gemäß Anspruch 1, dadurch gekennzeichnet, daß man ein Hydrochinon der Formel

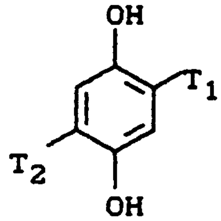

worin
$T_1$, $T_2$ unabhängig voneinander H, Halogen (Cl, Br) $SO_3H$, COOH, COOR (R = $C_1$-$C_4$-Alkyl), $C_1$-$C_4$-Alkoxy, $C_1$-$C_4$-Alkyl, Phenyl, Phenoxy bedeuten
und worin die Alkyl-, Alkoxy-, Phenyl- und Phenoxyreste weitersubstituiert sein können mit dem Amin der Formel

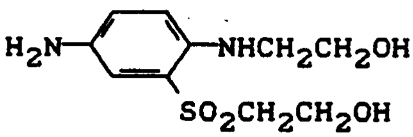

umsetzt.

**Claims**

1. Process for the preparation of 2,5-bis-(aminoarylamino)benzoquinones, characterised in that 2 moles of an aminoarylamine are reacted with 1 mole of a benzoquinone or hydroquinone in the presence of oxidants.

2. Process according to Claim 1, characterised in that the reaction is carried out in aqueous or aqueous/organic medium at about 10 to 120 ° C.

3. Process according to Claim 1, characterised in that the reaction is carried out in aqueous/acidic medium.

4. Process according to Claim 3, characterised in that the reaction is carried out in the presence of $H_2SO_4$, $H_3PO_4$, HCl or acetic acid.

5. Process according to Claim 1, characterised in that the oxidation is carried out using chlorates, bromates or iodates, optionally in the presence of vanadium compounds.

6. Process according to Claim 1, characterised in that the reaction is carried out with air in the presence of catalysts, in particular iron compounds, copper compounds, manganese compounds or vanadium compounds or anthraquinone-2-sulphonic acid.

7. Process according to Claim 1, characterised in that a hydroquinone of the formula

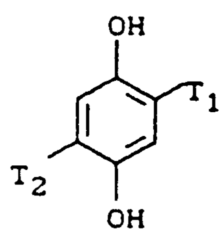

wherein
$T_1$ and $T_2$ independently of one another denote H, halogen (Cl, Br) $SO_3H$, COOH, COOR (R = $C_1$-$C_4$-

alkyl), $C_1$-$C_4$-alkoxy, $C_1$-$C_4$-alkyl, phenyl or phenoxy,
and wherein the alkyl, alkoxy, phenyl and phenoxy radicals can be further substituted, is reacted with the amine of the formula

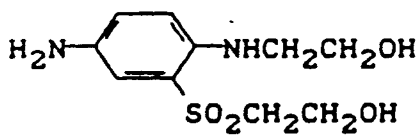

**Revendications**

1. Procédé de préparation de 2,5-bis-(aminoarylamino)-benzoquinones, caractérisé en ce que l'on fait réagir 2 mol d'une aminoarylamine avec 1 mol d'une benzoquinone ou d'une hydroquinone en présence d'agents oxydants.

2. Procédé selon la revendication 1, caractérisé en ce que l'on opère en milieu aqueux ou hydro-organique à des températures d'environ 10 à 120 ° C.

3. Procédé selon la revendication 1, caractérisé en ce que l'on opère en milieu aqueux acide.

4. Procédé selon la revendication 3, caractérisé en ce que l'on opère en présence d'$H_2SO_4$, de $H_3PO_4$, d'HCl ou d'acide acétique.

5. Procédé selon la revendication 1, caractérisé en ce que l'on oxyde par des chlorates, des bromates ou des iodates éventuellement en présence de dérivés du vanadium.

6. Procédé selon la revendication 1, caractérisé en ce que l'on opère avec de l'air en présence de catalyseurs, en particulier des dérivés du fer, du cuivre, du manganèse ou du vanadium ou l'acide anthraquinone-2-sulfonique.

7. Procédé selon la revendication 1, caractérisé en ce que l'on fait réagir une hydroquinone de formule

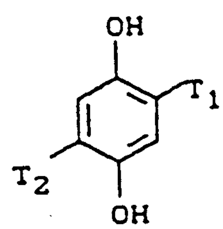

dans laquelle
$T_1$, $T_2$ représentent chacun, indépendamment l'un de l'autre, H, un halogène (Cl, Br), un groupe $SO_3H$, COOH, COOR (R = alkyle en $C_1$-$C_4$), alcoxy en $C_1$-$C_4$, alkyle en $C_1$-$C_4$, phényle, phénoxy,
les groupes alkyle, alcoxy, phényle et phénoxy pouvant porter d'autres substituants avec l'amine de formule